(19) Europäisches Patentamt
European Patent Office
Office européen des brevets

(11) Publication number : 0 554 004 A2

(12) **EUROPEAN PATENT APPLICATION**

(21) Application number : 93300415.2

(22) Date of filing : 21.01.93

(51) Int. Cl.$^5$ : **C07D 501/46**, A61K 31/545

(30) Priority : 27.01.92 JP 12221/92

(43) Date of publication of application : 04.08.93 Bulletin 93/31

(84) Designated Contracting States : AT BE CH DE DK ES FR GB IT LI NL SE

(71) Applicant : KATAYAMA SEIYAKUSYO CO. Ltd.
202, 2-1-15, Himesato, Nishiyodogawa-ku
Osaka-shi, Osaka-fu (JP)

(71) Applicant : Ajinomoto Co., Ltd.
15-1, Kyobashi 1-chome, Chuo-ku
Tokyo (JP)

(72) Inventor : Hayashi, Sadao
6-21 Shimuzu-cho,Ashiya-shi
Hyogo-ken (JP)
Inventor : Nakanishi, Eiji
c/o Centr. Res. Lab. Ajinomoto Co. Inc. of N0 1-1
Suzuki-cho Kawasaki-ku Kanagawa-ken (JP)
Inventor : Okunishi, Masahiko
c/o Cent. Res. Lab. Ajinomoto Co.Inc. of No1-1
Suzuki-cho, Kawasaki-ku Kanagawa-ken (JP)
Inventor : Kurita Yasuyuki
1-20-10 Kuzuhamentori-cho
Hirakata-shi, Osaka-fu (JP)
Inventor : Mizutani Akihito
1-B42-101 Otokoyamaishishiro
Yawata-shi, Kyoto-fu (JP)

(74) Representative : Nicholls, Kathryn Margaret et al
Mewburn Ellis, 2 Cursitor Street
London EC4A 1BQ (GB)

(54) **Cephem compounds and antibacterial agents.**

(57) Novel cephem compounds represented by the following general formula (I), and antibacterial agents containing at least one of such cephem compounds as an active ingredient are disclosed.

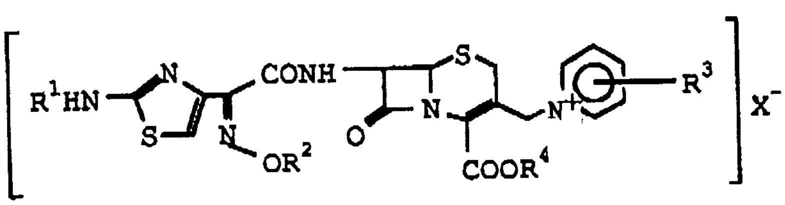

(I)

EP 0 554 004 A2

The present invention relates to novel cephem compounds which have a substituted pyridiniomethyl group at the 3-position, and also to antibacterial agents containing at least one of said compounds as an active principle and having an anti-MRSA activity. The term MRSA used in the present specification is an abbreviation of methicillin-resistant Staphylococcus aureus.

While various antibiotics have been used widely for the treatment of diseases, there have come out some pathogenic bacteria resistant to these antibiotics, such bacteria being called resistant bacteria or multipledrug resistant bacteria.

Among these multipledrug resistant bacteria, MRSA is much feared as a possible cause of intractable infection or hospital infection. Several anti-MRSA antibacterial agents have been developed, but request is still strong for the development of more effective novel anti-MRSA antibacterial agents.

Accordingly, it is desirable that embodiments of the present invention provide an antibacterial agent, especically one which is effective against MRSA.

The present invention thus provides novel antibacterial agents and novel cephem compounds usable as an active principle of such antibacterial agents

In an aspect of the present invention there are provided some novel cephem compounds. In another aspect of the present invention, there are provided some novel antibacterial agents containing, as an active ingredient, at least one of such cephem compounds.

In the accompanying drawings:

Fig. 1 shows the reaction scheme in Example 1;

Fig. 2 shows the reaction scheme in Example 2;

Fig. 3 shows the reaction scheme in Example 3;

Fig. 4 shows the reaction scheme in Example 4;

Fig. 5 shows the reaction scheme in Example 5;

The present invention pertains to the novel cephem compounds and the antibacterial agents, especially those effective against MRSA, which contain at least one of such novel cephem compounds as an active ingredient.

The present invention will be described in detail below.

In a first embodiment, the present invention provides novel cephem compounds represented by the following general formula (I):

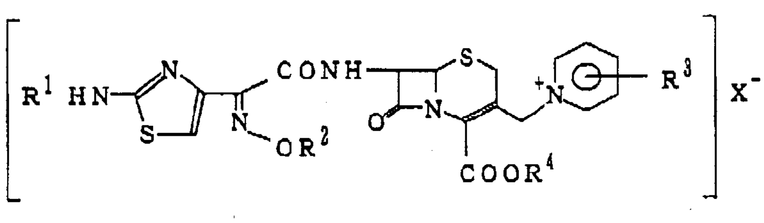

(I)

wherein $R^1$ is a hydrogen atom or a protective group of the amino group (i.e., an amino-protective group); $R^2$ is a hydrogen atom or a protective group of the hydroxyimino group (i.e., a hydroxyimino-protective group); $R^3$ is an alkyl group which may have appropriate substituent(s), a hydroxyl group which may have an appropriate substituent, a mercapto group which may have appropriate substituent(s), a cyano group, an acetyl group, an amino group which may have appropriate substituent(s), a carbamoyl group which may have appropriate substituent(s), an ureido group or a heterocyclic group which may have appropriate substituent(s); $R^4$ is a hydrogen atom, a protective group of the carboxyl group (i.e., carboxyl-protective group) or an anion (when $R^4$ is an anion, $X^-$ is excluded), and $X^-$ denotes the anion of an inorganic or organic acid (i.e., acid radical anion), said $R^3$ being preferably represented by the following formula (1) or (2):

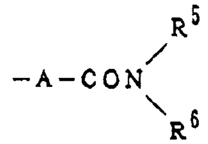

(1)

wherein A denotes a single bond, $-CH_2-$, -O-, -S-, -NH- or the like, and $R^5$ and $R^6$ represent independently a hydrogen atom or an alkyl group;

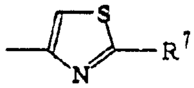

(2)

wherein $R^7$ is an alkyl group or an amino group which may have appropriate substituent(s).

In the above formula (I), $X^-$ represents 1/n of an anion having a valency of n (n being a positive integer), such as $Cl^-$, $NO_3^-$, 1/2 $SO_4^{--}$, $CF_3CO_2^-$, $CCl_3CO_2^-$, $CH_3CO_2^-$, 1/2 $^-O_2CCH(OH)CH(OH)CO_2^-$ (tartarate anion), $CH_3C_6H_4SO_3^-$(p-toluenesulfonate anion), $CH_3SO_3^-$ or the like.

Thus, the compounds of the present invention are novel cephem compounds in which the pyridiniomethyl group having a substituent is a 3-substituent group.

The compounds of the present invention represented by the general formula (I) can be prepared by a per se known method. For example, compounds represented by the following formula (Ia) belonging to the compounds of the present invention can be prepared by reacting a compound represented by the following formula (II) with NaI to form an iodide, then reacting it with a pyridine derivative having a substituent represented by the following formula (III) to form a compound having a pyridinio group represented by the following formula (IV), and finally removing the protective group.

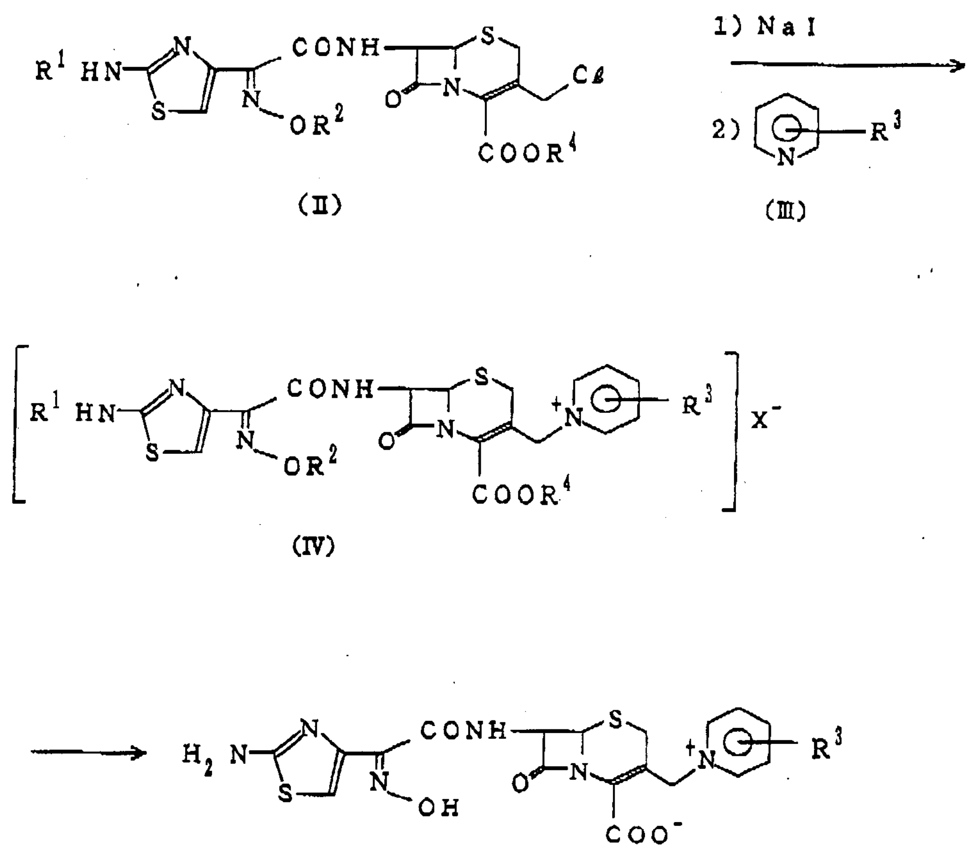


(II)

(III)

(IV)

(I a)

In the above formulae, $R^1$, $R^2$, $R^3$ and $R^4$ represent the same meaning as defined before. The protective group of the protected amino group is one which is generally used in the chemistry of cephalosporin derivatives and easy to eliminate, that is, a protective group which can be eliminated under relatively mild conditions, such as a formyl, t-butoxycarbonyl, benzyloxycarbonyl, p-nitrobenzyloxycarbonyl, trityl or the like group. The protective group of the protected hydroxyimino group is, for instance, a formyl, chloroacetyl, benzoyl, p-nitrobenzoyl, β,β,β-trichloroethoxycarbonyl, tetrahydropyranyl, trityl or the like group. These can be easily removed under a per se known reaction condition. And, the protective group of the protected carboxyl group is, for example, β,β,β-trichloroethyl, p-methoxybenzyl, p-nitrobenzyl, benzhydryl or the like group, and these can be easily eliminated.

In the above reaction, the compounds can be used in their salt form. Such salts include hydrochlorides, nitrates, sulfates, acetates, trifluoro-acetates, trichloroacetates, tartarates, p-toluenesulfonates, methanesulfonates and the like.

Compounds of the present invention represented by the formula (Ia) can be also obtained by reacting a compound represented by the following formula (V) with a compound represented by the following formula (VI) and, if necessary, removing the protective group.

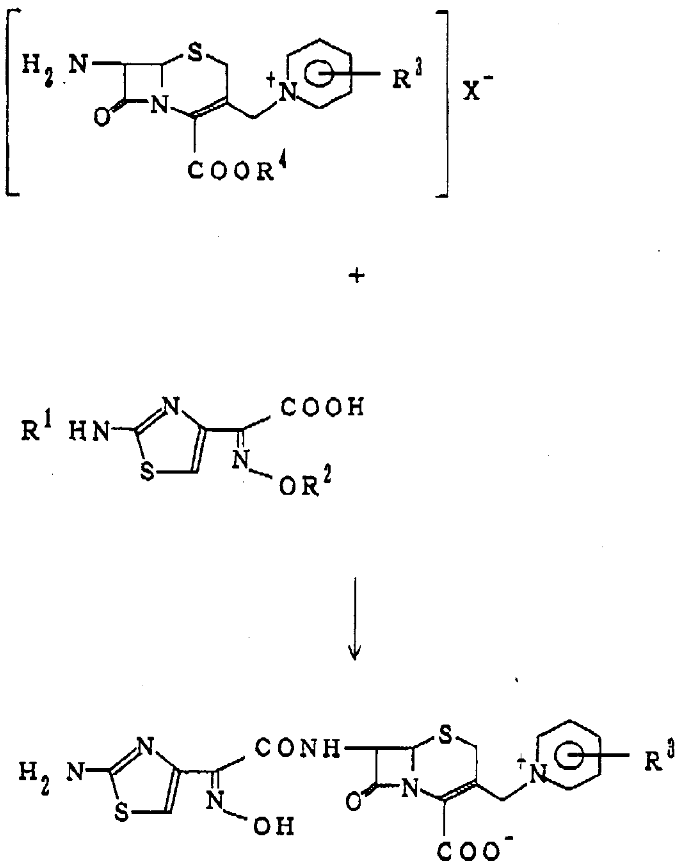

(V)

(VI)

(I a)

wherein $R^3$, $R^4$, $R^1$ and $R^2$ represent the same meaning as defined before.

The compounds represented by the formula (Ia) can be in their salt form, and can be applied in such form for, e.g., medicinal preparations. Such salts include hydrochlorides, nitrates, sulfates, acetates, trifluoroacetates, trichloroacetates, tartarates, p-toluenesulfonates, methanesulfonates and the like.

Incidentally, in the compounds represented by the above-shown general formula (I), the structure of the 2-aminothiazole group portion is in the equilibrium state between the two tautomers shown by the following equilibrium formula:

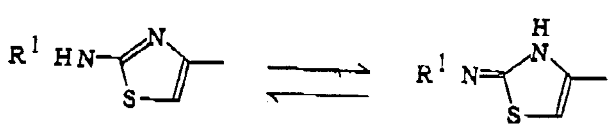

(A) (A)′

It is well known that both of the tautomers have an equilibrium relation with each other and are interconvertible each from the other. These isomers can be substantially regarded as one and the same compound. So, in the present specification, for convenience's sake, the desired compounds as well as their starting compounds both containing the groups of said tautomers, are described as those having one of the tautomers,

namely those having the 2-aminothiazole group expressed by the formula (A).

In a second embodiment, the present invention provides novel antibacterial agents containing a cephem compound of the formula (I) as an active ingredient.

The novel cephem compounds of the present invention, as will be seen from the Test Examples given hereinafter, show a strong wide-ranging antibacterial spectrum and have a remarkable antibacterial action, especially against MRSA. When these compounds are used in the form of their salt for preparing an antibacterial agent, such a salt needs of course to be physiologlcally acceptable one.

The novel cephem compounds of the present invention can be worked into the commonly used forms of medicinal preparations by mixing one or more of the compounds with a pharmaceutically acceptable carrier such as an organic or inorganic solid or liquid excipient suited for the desired way of administration such as oral, parenteral or external. The medicinal preparations may be in the solid form such as tablet, granules, powder, capsule, or the like, or in the liquid form such as solution, suspension, syrup, emulsion or the like. If necessary, auxiliary substance(s), stabilizer, wetting agent and other ordinarily used additives may be incorporated into the medicinal preparations. Thus, the ways of administration of the medicinal preparations can be in accordance with those of the known antibacterial agents.

The dose of the cephem compounds of the present invention varies depending on type of the preparation, age of the patient, kind of the disease to be treated and other factors, but generally it is about 5 to 3,000 mg or more per day for an adult patient with a body weight of about 60 kg. It may be administered in several portions in a day. One portion, in this case, is about 1 to 1,000 mg.

The antibacterial agents of the present invention, when tested, showed no acute toxicity.

## Examples

The present invention will be further explained below with reference to some synthesis examples and test examples.

### Example 1

Synthesis of 7β-[2-(2-aminothiazol-4-yl)-2-syn-hydroxyiminoacetamido]-3-[(3-carbamoyl-1-pyridinio)methyl]-3-cephem-4-carboxylic acid chloride (Compound (1))

The reaction scheme in this example is shown in Fig. 1.

(i) Synthesis of Compound (1c) (p-methoxybenzyl 7β-[2-(2-tritylaminothiazol-4-yl)-2-syn-trityloxyiminoacetamido]-3-chloromethyl-3-cephem-4-carboxylate)

4.72 g or the hydrochloride of p-methoxybenzyl 7-amino-3-chloromethyl-3-cephem-4-carboxylate was dissolved in 300 ml of ethyl acetate and washed with an aqueous solution of $NaHCO_3$ to form the free amine, and the latter was dried over $Na_2SO_4$ and concontrated to obtain 6.90 g of Compound (1b). This compound was dissolved in 245 ml of methylene chloride, added with 12.294 g of Compound (1a) (2-(2-tritylaminothiazol-4-yl)-2-syntrityloxyiminoacetic acid), followed by further addition of 4.151 g of DCC, then stirred at room temperature for 1.5 hours and concentrated. The residue was dissolved in ethyl acetate and, after filtering off the insolubles, concentrated. The residue was subjected to chromatography on a column of silica gel (100 g) with a 10:1 mixture of benzene and ethyl acetate as the developing solvent to obtain 11.33 g of Compound (1c) in a yield of 60.5%.

The identification data of the compound were as follows.

IR$\nu_{max}$ $^{KBr}$ $cm^{-1}$: 1791, 1723, 1686, 1514, 1491, 1446, 1243, 1173, 1160, 752, 700.

NMRδ($CDCl_3$) ppm: 3.20, 3.46 (2H, ABq, J = 18), 3.72 (3H, s), 4.30, 4.44 (2H, ABq, J = 12), 4.91 (1H, d, J = 4.5), 5.14 (2H, s), 5.91 (1H, d.d, J = 4.5,9) 6.31 (1H, s), 5.78 (2H, d, J = 9), 6.94-7.51 (33H, m).

(ii) Synthesis of Compound (1d) (p-methoxybenzyl 7β-[2-(2-tritylaminothiazol-4-yl)-2-syn-trityloxyiminoacetamido]-3-[(3-carbamoyl-1-pyridinio)methyl]-3-caphem-4-carboxylate iodide)

1.269 g of Compound (1c) was dissolved in 30 ml of dry acetone, added with 280 mg of NaI and stirred at room temperature for 45 minutes. Then the solvent was distilled away, and the residue was dissolved in ethyl acetate, washed with water and dried over $Na_2SO_4$. Thereafter, the solvent was distilled away, and the residue was dissolved in 35 ml of THF and added with 227 mg of nicotinic acid amide with stirring under ice cooling. The mixture was stirred at the same temperature for 30 minutes and then at room temperature for 6.5 hours, dissolved in ethyl acetate, washed with a saline solution and dried over $Na_2SO_4$. Then the solvent was

distilled away, and the residue was subjcted to silica gel (65 g) column chromatography with chloroform/methanol (9/1) as the developing solvent to obtain 845 mg of Compound (1d) in a yield of 55%.

The identification data of the compond were as follows.

IR$\nu_{max}$ $^{Nujol}$ $cm^{-1}$: 3348, 3173, 1790, 1678, 1611, 1247, 1173, 1096, 1029, 960, 826, 751, 700.

(iii) Synthesis of Compound (1)

510 mg of Compound (1d) was dissolved in 3 ml of methylene chloride, and the solution was added dropwise with 0.5 ml of anisole and 1.1 ml of trifluoroacetic acid with stirring under ice cooling, followed by further stirring at the same temperature for 15 minutes and then at room temperature for 2 hours. Then the solvent was distilled away, and the residue was added dropwise with 2.1 ml of trifluoroacetic acid and 0.5 ml of water with stirring under ice cooling, and the mixture was further stirred at the same temperature for 15 minutes and then at room temperature for 2 hours. Thereafter, the solvent was distilled away, and the residue was solidified by adding ether, filtered out, washed with ether and dried. The resulting product was dissolved in a hydrochloric acid-methanol solution, passed through a column of 100 ml of "HP-20" (polystyrene resin manufactured by Mitsubishi Chemical Industries Co., Ltd.) for development with water containing 10% of methanol, and lyophilized to obtain 50 mg of Compound (1) (28% yield).

The identification data of the compound were as follows.

IR$\nu_{max}$ $^{Nujol}$ $cm^{-1}$: 3297, 3179, 1771, 1673, 1609, 1528, 1182, 1061, 972, 815, 722.

NMR$\delta$(DMSO-$d_6$) ppm: 3.54 (2H, br. s), 5.17 (1H, d, J = 4.5), 5.63 (2H, br. s), 5.80 (1H, d.d, J = 4.5, 9), 6.73 (1H, s), 7.9-8.1 (1H, m), 8.6-9.2 (2H, m), 9.51 (1H, br. s).

Example 2

Synthesis of 7β-[2-(2-aminothiazol-4-yl)-2-syn-hydroxyiminoacetamido]-3-[(3-acetyl-1-pyridinio)methyl]-3-cephem-4-carboxylic acid chloride (Compound (2))

The reaction scheme in this example is shown in Fig. 2.

(i) Synthesis of Compound (2a) (p-methoxybenzyl 7β-[2-tritylaminothiazol-4-yl)-2-syn-hydroxyiminoacetamido]-3-[(3-acetyl-1-pyridinio)methyl]-3-cephem-4-carboxylate iodide)

1.02 g of Compound (1c) was dissolved in 25 ml of acetone, added with 270 mg of NaI and stirred under ice cooling for 10 minutes and then at room temperature for 45 minutes. Then the solvent was distilled away, the residue was extracted with ethyl acetate, and the extract was washed with water and dried over $Na_2SO_4$. Thereafter, the solvent was distilled away, and the residue was dissolved in 30 ml of THF, added with 168 μl of 3-acetylpyridine with stirring under ice cooling, followed by further stirring at the same temperature for one hour and then at room temperature for 6 hours, and then extracted with chloroform. The extract was washed with water and dried over $Na_2SO_4$. Thereafter, the solvent was distilled away, and the residue was subjected to chromatography on a column of 60 g of silica gel with methanol/chloroform (15:85) as the developing solvent to obtain 415 mg of Compound (2a) (34% yield).

The identification data of the compound were as follows.

IR$\nu_{max}$ $^{Nujol}$ $cm^{-1}$: 3373, 1789, 1701, 1626, 1612, 1586.

NMR $\delta$($CDCl_3$) ppm: 2.68 (3H, s), 3.55, 3.85 (2H, ABq, J = 18), 3.67(3H, s), 5.07 (1H, d, J = 4.5), 5.13 (2H, s), 5.76, 6.04 (2H, ABq, J = 14), 5.87 (1H, d.d, J = 4.5, 9), 6.30 (1H, s), 6.66 (2H, d J = 9), 6.8-7.6 (34H, m), 7.82 (1H, br. d.d, J = 6, 8), 8.55 (1H, br. d, J = 8), 9.13 (1H, br. d, J = 6), 9.71 (1H, br. s).

(ii) Synthesis of Compound (2)

350 mg of Compound (2a) was dissolved in 5 ml of dichloromethane, and the solution was added with 0.4 ml of anisole and then with 0.8 ml of trifluoroacetic acid with stirring under ice cooling, followed by stirring at the same temperature for 15 minutes and then at room temperature for one hour. The solvent was distilled away, and the residue was added with 0.8 ml of trifluoroacetic acid and 0.4 ml of water and stirred at room temperature for 2.5 hours. Then the solvent was distilled away, and the residue was treated with ether, filtered out and washed with ether. The resulting product was dissolved in a mixed solution of hydrochloric acid and methanol, subjected to chromatography through a column of "HP-20" (60 ml) for development with water and then with water containing 15% of methanol, and lyophilized to obtain 71 mg of Compound (2) (50% yield).

The identification data of Compound (2) were as follows.

IR$\nu_{max}$ $^{Nujol}$ $cm^{-1}$: 3289, 3169, 1775, 1698, 1629, 1607, 1528.

NMR δ(DMSO-$d_6$ + $CD_3OD$) ppm: 2.7 (3H, S), 3.29, 3.55 (2H, ABq, J = 18), 5.09 (1H, d, J = 4.5), 5.44, 5.71 (2H, ABq, J = 15), 5.73 (1H, d, J = 4.5), 6.56 (1H, s), 8.13 (1H, br. d.d, J = 6, 8), 8.86 (1H, br. d, J = 8), 9.29 (1H, br. d, J = 6), 9.62 (1H, br. s).

Example 3

Synthesis of 7β-[2-(2-aminothiazol-4-yl)-2-syn-hydroxyiminoacetamido]-3-[(3-amino-1-pyridinio)methyl]-3-cephem-4-carboxylate hydrochloride (Compound (3))

The reaction scheme in this example is shown in Fig. 3.

(i) Synthesis of Compound (3b) (p-methoxybenzyl 7β-[2-(2-tritylaminothiazol-4-yl)-2-syn-trityloxyiminoacetamido]-3-[3(di-t-butyloxycarbonyl)amino-1-pyridinio)methyl]-3-cephem-4-carboxylate iodide)

1.02 g of Compound (1c) was dissolved in 25 ml of acetone, and the solution was added with 300 mg of NaI and stirred at room temperature for 45 minutes. Then the solvent was distilled away, and the residue was dissolved in ethyl acetate and dried over $Na_2SO_4$. The solvent was distilled away, and the residue was dissolved in 30 ml of THF, added with 382 mg of Compound (3a) (3-(di-t-butyloxycarbonylamino)pyridine) and stirred under ice cooling for one hour and then at room temperature for another one hour, after which the solvent was distilled away, and the residue was dissolved in chloroform, washed with water and dried over $Na_2SO_4$. Then the solvent was distilled away, and the residue was subjected to chromatographic development through a column of 60 g of silica gel by using a 15:85 mixed solvent of methanol and chloroform as the developer. The developing solution containing the desired compound was concentrated to obtain 912 mg of Compound (3b) (65% yield).

The identification data of the compound were as follows.

IR$\nu_{max}$ $^{Nujol}$ $cm^{-1}$: 3358, 1791, 1718, 1690, 1612, 1512, 1491, 1243, 1174, 1151, 1114, 1030, 962, 844, 827, 753, 701.

(ii) Synthesis of Compound (3)

912 mg of Compound (3b) was dissolved in 5 ml of dichloromethane, added with 0.8 ml of anisole and then with 2.5 ml of trifluoroacetic acid with stirring under ice cooling, followed by further stirring at the same temperature for 20 minutes and then at room temperature for 30 minutes. The solvent was distilled away, and the residue was added with 3 ml of trifluoroacetic acid and then with 1.2 ml of water with stirring under ice cooling, and stirred at the same temperature for 30 minutes and then at room temperature for 2.4 hours. The solvent was distilled away, the residue was treated with isopropyl ether to be solidified, and the solid was filtered out, washed with isopropyl ether and dried. The resulting product was dissolved in a hydrochloric acid/methanol solution, subjected to chromatographic development through a column of 100 ml of "HP-20" by using water containing 10-15% of methanol as the developing solvent, and lyophilized to obtain 48 mg of Compound (3) (13% yield).

The identification data of the compound were as follows.

IR$\nu_{max}$ $^{Nujol}$ $cm^{-1}$: 3338, 3207, 1769, 1608, 1530, 1509, 1192, 1153, 1041, 984, 796, 752, 721, 675.

NMR δ(DMSO-$d_6$) ppm: 3.31, 3.62 (2H, ABq, J = 18), 5.18 (1H, d, J = 4.5), 5.27, 5.46 (2H, ABq, J = 14), 5.83 (1H, d.d, J = 4.5, 9), 6.77 (1H, s), 7.3-7.8 (2H, m), 7.8-8.2 (2H, m), 9.53 (1H, d, J = 9).

Example 4

Synthesis of 7β-[2-(2-aminothiazol-4-yl)-2-syn-hydroxyiminoacetamido]-3-[(3-ureido-1-pyridinio)methyl]-3-cephem-4-carboxylic acid chloride (Compound (4))

The reaction scheme in this example is shown in Fig.4.

(i) Synthesis of Compound (4b) (p-methoxybenzyl 7β-[2-(2-tritylaminothiazol-4-yl)-2-syn-trityloxyiminoacetamido]-3-[(3-ureido-1-pyridinio)methyl]-3-cephem-4-carboxylate iodide)

733 mg of Compound (1c) was dissolved in 23 ml of acetone, and the solution was added with 215 mg of NaI and stirred at room temperature for one hour. The solvent was distilled away, the residue was extracted with ethyl acetate, and the extract was washed with a saline solution and dried over $Na_2SO_4$. Then the solvent was distilled away, and the residue was added with 148 mg of Compound (4a) (3-ureidopyridine), dissolved

in 15 ml of THF, and stirred for 3 hours and 50 minutes. The solution was extracted with ethyl acetate, and the extract was washed with a saline solution and dried over $Na_2SO_4$. Then the solvent was distilled away, and the residue was subjected to chromatography on a column of silica gel (60 g) using chloroform containing 15% of methanol as the developing solvent to obtain 345 mg of Compound (4b) (38% yield).

The identification data of the compound were as follows.

$IR\nu_{max}^{Nujol}$ $cm^{-1}$: 3377, 3202, 1788, 1675, 1602, 1583.

NMR $\delta(CDCl_3)$ ppm: 3.23, 3.57 (2H, ABq, J = 18), 3.68 (3H, s), 5.21 (2H, s), 5.16 (1H, d, J = 4.5), 5.68, 5.95 (2H, ABq, J = 14), 5.80 (1H, d.d, J = 9, 4.5), 6.45 (1H, s), 6.7-9.3 (38H, m), 9.5-9.9 (2H, m).

(ii) Synthesis of Compound (4)

345 mg of Compound (4b) was dissolved in 3 ml of dry dichloromethane, added dropwise with 0.3 ml of anisole and then with 0.7 ml of trifluoroacetic acid with stirring under ice cooling, and stirred at the same temperature for 10 minutes and then at room temperature for 45 minutes. The solvent was distilled away, and the residue was added with 2 ml of trifluoroacetic acid and then with 1 ml of water with stirring under ice cooling, followed by further stirring at the same temperature for 30 minutes and then at room temperature for 2.5 hours. Thereafter, the solvent was distilled away, and the residue was treated with ether, filtered out and washed with ether. The resulting product was dissolved in a hydrochloric acid/methanol solution, subjected to chromatographic development through a column of 60 ml of "HP-20" with water containing 10% of methanol as the developing solvent, and lyophilized to give 81 mg of Compound (4) (46% yield).

The identification data of the compound were as follows.

$IR\nu_{max}^{Nujol}$ $cm^{-1}$: 3272, 3190, 3072, 1774, 1679, 1629.

NMR $\delta(DMSO\text{-}d_6 + CD_3OD)$ ppm: 3.35, 3.54 (2H, ABq, J = 18), 5.10 (1H, d, J = 4.5), 5.33 (2H, br. s), 5.73 (1H, d, J = 4.5), 6.68 (1H, s), 7.6-8.5 (3H, m), 9.07 (1H, m).

Example 5

Synthesis of 7β-[2-(2-aminothiazol-4-yl)-2-syn-hydroxyiminoacetamido]-3-[[4-(2-aminothiazol-4-yl)-1-pyridinio]methyl]-3-cephem-4-carboxylic acid chloride (Compound (5))

The reaction scheme in this example is shown in Fig. 5.

(i) Synthesis of Compound (5b) (p-methoxybenzyl 7β-[2-(2-tritylaminothiazol-4-yl)-2-syn-trityloxyiminoacetamido]-3-[[4-(2-aminothiazol-4-yl)-1-pyridinio]methyl]-3-cephem-4-carboxylate iodide)

867 mg of Compound (1c) was dissolved in 20 ml of dry acetone, and the solution was added with 255 mg of NaI, stirred at room temperature for 45 minutes, and extracted with ethyl acetate. The extract was washed with a saline solution and dried over $Na_2SO_4$. Then the solvent was distilled away, and the residue was added with 463 mg of Compound (5a) (4-(2-aminothiazol-4-yl)pyridine), dissolved in 11 ml of THF, stirred for 3 hours and 50 minutes, and extracted with ethyl acetate. The extract was washed with a saline solution and dried over $Na_2SO_4$, The solvent was distilled away, and the residue was subjected to chromatography on a column of silica gel (60 g ) using chloroform containing 15% of methanol as the developing solvent. The process gave 833 mg of Compound (5b) in a yield of 76 %.

The identification data of the compound were as follows.

$IR\nu_{max}^{Nujol}$ $cm^{-1}$: 3377, 1786, 1715, 1677, 1630.

NMR $\delta(CDCl_3)$ ppm: 3.67 (3H, s), 3.03, 3.80 (2H, ABq, J = 18), 5.02 (1H, d, J = 4.5), 5.15 (2H, s), 5.24, 5.68 (2H, ABq, J = 14), 5.87 (1H, d.d, J = 9, 4.5), 6.30 (1H, s), 6.5-7.8 (35H, m), 7.95, 8.93 (4H, ABq, J = 6), 8.27 (1H, d, J = 9).

(ii) Synthesis of Compound (5)

1.09 g of Compound (5b) was dissolved in 3.6 ml of dry dichloromethane, and the solution was added with 0.9 ml of anisole and then with 2 ml of trifluoroacetic acid with stirring under ice cooling and stirred at the same temperature for 10 minutes and then at room temperature for 30 minutes. The solvent was distilled away, and the residue was added dropwise with 3 ml of trifluoroacetic acid and then with 1.4 ml of water with stirring under ice cooling, followed by further stirring at the same temperature for 10 minutes and then at room temperature for 2.5 hours. Then the solvent was distilled away, and the residue was treated with ether, filtered out, dissolved in a hydrochloric acid/methanol solution, subjected to chromatography through a column of 60 ml of "HP-20" by using water containing 10% of methanol as the developing solvent, and lyophilized to obtain

348 mg of Compound (5) (69% yield).

The identification data of the compound were as follows.

IR$\nu_{max}$ $^{Nujol}$ $cm^{-1}$: 3253 (sh), 1774, 1630, 1533.

NMR $\delta$(DMSO-$d_6$ + $CD_3OD$) ppm: 3.36, 3.64 (2H, ABq, J = 18), 5.19 (1H, d, J = 4.5), 5.37, 5.53 (2H, ABq, J = 14), 5.81 (1H, d, J = 4.5), 6.82 (1H, s), 7.94 (1H, s), 8.27, 8,79 (4H, ABq, J = 7).

Test Example 1 (Test on antibacterial potency against standard strains)

The minimum growth inhibitory concentration (MIC, μg/ml) of 5 compounds synthesized in Examples 1 to 5 and 2 control agents for each of the 20 standard laboratory strains (standard strains) shown in Table 1 was determined according to the standard method of the Japan Chemotherapical Society (Chemotherapy, 29, 76-79 (1981) under "Re-revision of the measuring methods of minimum growth inhibitory concentration (MIC)") as described below.

By using each test strain cultured overnight at 37°C in a Muller-Hinton broth, there was prepared a bacterial solution with a bacterial cell population of 1 x $10^6$ CFU/ml of the same bouillon. The bacterial solution was inoculated into the Muller-Hinton agar media each containing respectively one test agent and, after overnight incubation at 37°C, the minimum concentration that inhibited growth of the bacteria was determined and shown as MIC also in Table 1.

Staphylococcus aureus 167 and Staphylococcus aureus 195 used in the test were MRSA. CTM (cefotiam) and FMOX (flomoxef) were used as control agents.

The results are shown in Table 1.

Table 1 : Antibacterial potency against standard strains (MIC: μg/ml)

| Bacterial strains tested | Test agents | | | | | | |
|---|---|---|---|---|---|---|---|
| | Compd. (1) | Compd. (2) | Compd. (3) | Compd. (4) | Compd. (5) | CTM | FMOX |
| Staphylococcus aureus 209P JC-1 | 0.2 | 0.2 | 0.2 | 0.1 | 0.2 | 0.2 | 0.39 |
| Staphylococcus aureus Smith | 0.39 | 0.39 | 0.39 | 0.2 | 0.2 | 0.39 | 0.39 |
| Staphylococcus aureus 167 | 6.25 | 12.5 | 12.5 | 12.5 | 6.25 | >100 | 100 |
| Staphylococcus aureus 195 | 3.13 | 6.25 | 3.13 | 3.13 | 3.13 | >100 | 6.25 |
| Staphylococcus epidermidis IAM 1296 | 0.1 | 0.2 | 0.1 | 0.05 | 0.1 | 0.2 | 0.2 |

| | | | | | | | |
|---|---|---|---|---|---|---|---|
| Micrococcus luteus ATCC 9341 | 0.05 | 0.1 | 0.05 | 0.39 | 0.025 | 0.39 | 0.2 |
| Bacillus subtilis ATCC 6633 | 0.39 | 0.78 | 0.2 | 0.2 | 0.2 | 0.2 | 0.2 |
| Escherichia coli NIHJ JC-2 | 0.39 | 0.39 | 0.2 | 0.1 | 0.2 | 0.1 | 0.05 |
| Escherichia coli CSH2 (RK1) | 0.78 | 0.78 | 0.39 | 0.78 | 0.2 | 0.2 | 0.05 |
| Escherichia coli CSH2 (RE45) | 0.39 | 0.78 | 0.2 | 0.39 | 0.05 | 0.2 | 0.1 |
| Klebsiella pneumoniae IFO 3317 | 0.2 | 0.39 | 0.1 | 0.1 | 0.05 | 0.1 | 0.05 |
| Klebsiella pneumoniae No. 42 | 0.78 | 0.78 | 0.2 | 0.39 | 0.1 | 0.39 | 0.1 |
| Proteus mirabilis IFO 3849 | 0.39 | 0.39 | 0.2 | 0.1 | 0.1 | 0.39 | 0.2 |
| Proteus vulgalis OX-19 | 0.78 | 0.39 | 0.39 | 0.78 | 0.2 | 0.39 | 0.39 |
| Morganella morganii IFO 3848 | 0.39 | 0.2 | 0.05 | 0.05 | 0.05 | 0.1 | 0.39 |
| Serratia marcescens IAM 1184 | 0.2 | 0.39 | 0.1 | 0.1 | 0.2 | 3.13 | 0.2 |
| Enterobacter cloacae ATCC 13407 | 0.78 | 1.56 | 3.13 | 0.39 | 0.78 | 50 | 25 |

| | | | | | | | |
|---|---|---|---|---|---|---|---|
| Citrobacter freundii ATCC 8090 | 0.39 | 0.39 | 0.2 | 0.1 | 0.2 | 0.78 | 0.39 |
| Pseudomonas aeruginosa NCTC 10490 | 3.13 | 3.13 | 3.13 | 1.56 | 1.56 | >100 | >100 |
| Psuedomonas aeruginosa IFO 3445 | 25 | 25 | 6.25 | 12.5 | 25 | >100 | >100 |

It can be understood from Table 1 that the novel cephem compounds of the present invention show a strong wide-ranging antibacterial spectrum.

Test Example 2 (Test on antibacterial potency against clinically isolated MRSA)

A test on the antibacterial potency against clinically isolated MRSA was conducted in the following way.

52 strains of clinically isolated MRSA were used, and MIC was determined for each of the strains according to the standard method of the Japan Chemotherapical Society as in Test Example 1. The minimum growth inhibitory concentration which inhibited growth of 50% of the strains tested was expressed as MIC50 (μg/ml), and the minimum growth inhibitory concentration which inhibited growth of 90% of the strains was expressed as MIC90 (μg/ml).

In this test, FMOX (flomoxef), IPM/CS (imipenem/cilastatin), CMZ (cefmetazole), MINO (minocycline) and DMPPC (methicillin) were used as control agents.

The results are shown in Table 2.

Table 2: Antibacterial potency against clinically isolated MRSA

| Test agents | Antibacterial potency | |
|---|---|---|
| | MIC50 | MIC90 |
| Compound (1) | 3.79 | 11.40 |
| Compound (2) | 3.77 | 9.47 |
| Compound (3) | 3.94 | 9.21 |
| Compound (4) | 2.17 | 6.16 |
| Compound (5) | 3.13 | 8.69 |
| FMOX | 8.16 | 40.21 |
| IPM/CS | 1.56 | 28.72 |
| CMZ | 16.8 | 49.8 |
| MINO | 0.2 | 1.5 |
| DMPPC | 42.5 | >100 |

It can be understood from Table 2 that the novel cephem compounds of the present invention have excellent antibacterial potency against MRSA.

Example 6

Synthesis of 7β-[2-(2-aminothiazol-4-l)-2-syn-hydroxyiminoacetamido]-3-[(3-N-methylcarbamoyl)-1-pyridinio)methyl]-3-cephem-4-carboxylic acid chloride (Compound (6))

(i) Synthesis of Compound (6b) (p-methoxybenzyl 7β-[2-(2-tritylaminothiazol-4-yl)-2-syn-trityloxyiminoacetamido]-3-[(3-N-methylcarbamoyl-1-pyridinio)methyl]-3-cephem-4-carboxylate iodide)

628 mg of Compound (1c) was dissolved in 20 ml of dry acetone, and the solution was added with 277 mg of NaI with stirring under ice cooling and stirred at the same temperature for 15 minutes and then at room temperature for 45 minutes. Then the solution was concentrated, extracted with ethyl acetate, washed with a saline solution and dried over $Na_2SO_4$. Thereafter, the solvent was distilled away and the residue was dissolved in 20 ml of acetonitrile and added with 83 mg of 3-(N-methylcarbamonyl)pyridine with stirring under ice cooling. The mixture was stirred at the same temperature for 15 minutes and then at room temperature for 6 hours, extracted with ethyl acetate, washed with a saline solution and dried over $Na_2SO_4$. Then, the solvent was distilled away and ether was added to the residue to cause separating of the crystals. The crystals were filtered out and washed with ether to obtain 640 mg of Compound (6b) (83% yield).

The identification data of Compound (6b) were as follows.

IR$\nu_{max}$ $^{Nujol}$ $cm^{-1}$: 3310, 1789, 1718, 1669, 1612, 1031, 961, 826, 729, 699.

NMR δ(DMSO-$d_6$) ppm: 2.88 (3H, d, J = 4.5), 3.50 (2H, br. s), 3.70 (3H, s), 5.02 (1H, d, J = 4.5), 5.33 (2H, s), 5.50, 5.66 (2H, ABq, J = 13), 5.76 (1H, d.d, J = 4.5, 9), 6.39 (1H, s), 6.72 (2H, d, J = 9), 6.6-7.7 (33H, m), 7.8-9.4 (5H, m), 9.66 (1H, d. J = 9).

(ii) Synthesis of Compound (6)

590 mg of Compound (6b) was treated in the same way as Example 1 (iii) to obtain 52 mg of Compound (6) (19% yield).

The identification data of Compound (6) were as as follows.

IR$\nu_{max}$ $^{Nujol}$ $cm^{-1}$: 3195, 3068, 1777, 1659, 1628, 1060, 1010, 803, 711, 675.

NMR δ(DMSO-$d_6$) ppm: 2.88 (3H, d. J = 2,5), 3.58 (2H, br. s), 5.23 (1H, d, J = 4.5), 5.64 (2H, br. s), 5.80 (1H, d.d, J = 4.5, 9), 6.79 (1H, s), 8.0-9.9 (4H + 1H, m).

Example 7

Synthesis of 7β-[2-(2-aminothiazol-4-yl)-2-syn-hydroxyiminoacetamido]-3-[(3-carbamoylmethyl-1-pyridinio)methyl]-3-cephem-4-carboxylic acid chloride (Compound (7))

(i) Synthesis of Compound (7b) (p-methoxybenzyl 7β-[2-(2-tritylaminothiazol-4-yl)-2-syn-trityloxyiminoacetamido]-3-[(3-carbamoylmethyl-1-pyridinio)methyl]-3-cephem-4-carboxylate iodide)

1.02 g of Compound (1c) was treated according to the same procedure as Example 6 (i) by using 450 mg of NaI and 177 mg of 3-carbamoylmethylpyridine to obtain 1.15 g of Compound (7b) (93% yield).

The identification data of Compound (7b) were as follows.

IR$\nu_{max}$ $^{Nujol}$ $cm^{-1}$: 3173, 3321, 1788, 1717, 1672, 1611, 1098, 1064, 1030, 963, 825, 752, 700, 633.

NMR δ(DMSO-$d_6$) ppm; 3.36 (4H, br. s), 3.70 (3H, s), 5.20 (2H + 1H, br. s), 5.56 (2H, br. s), 5.84 (1H, d.d, J = 4.5, 9), 6.44 (1H, s), 6.6-7.7 (35H, m), 7.8-9.0 (4H, m), 8.5 (2H, br. s), 9.73 (1H, d, J = 9).

(ii) Synthesis of Compound (7)

1.00 g of Compound (7b) was subjected to the same treatments as in Example 1 (iii) to obtain 110 mg of Compound (7) (20% yield).

The identification data of Compound (7) were as follows.

IR$\nu_{max}$ $^{Nujol}$ $cm^{-1}$: 3300 (sh), 3160, 1774, 1660, 1626, 1059, 1011, 806, 721, 680.

NMR δ(DMSO-$d_6$) ppm: 3.37, 3.52 (2H, ABq, J = 19), 3.37 (2H, s), 5.15 (1H, d, J = 4.5), 5.48, 5.61 (2H, ABq, J = 10), 5.75 (1H, d.d, J = 4.5, 9), 6.72 (1H, s), 8.06 (1H, m), 8.43 (1H, m), 8.86 (1H, m), 8.94 (1H, br. s), 9.52 (1H, d, J = 9).

Example 8

Synthesis of 7β-[2-(2-aminothiazol-4-yl)-2-syn-hydroxyiminoacetamido]-3-[(3-carbamoyloxy-1-pyridinio)methyl]-3-cephem-4-carboxylic acid chloride (Compound (8))

(i) Synthesis of Compound (8b) (p-methoxybenzyl 7β-[2-(2-tritylaminothiazol-4-yl)-2-syn-trityloxyiminoacetamido]-3-[(3-carbamoyloxy-1-pyridinio)methyl]-3-cephem-4-carboxylate iodide)

500 mg of Compound (1c) was subjected to the same treatments as in Example 6 (i) by using 220 mg of NaI and 68 mg of 3-carbamoyloxypyridine to obtain 540 mg of Compound (8b) (88% yield).

The identification data of Compound (8b) were as follows.

$IR\nu_{max}^{Nujol}$ $cm^{-1}$: 3359, 1787, 1670, 1611, 1583, 1033, 964, 918, 826, 748, 698.

NMR δ($CDCl_3$ + $CD_3OD$) ppm: 3.74, (5H, br. s), 5.05 (1H, d, J = 4.5), 5.16 (2H, s), 5.40, 5.53 (2H, ABq, J = 13), 5.87 (1H, d, J = 4.5), 6.40 (1H, s), 6.77 (1H, d, J = 8.5), 6.6-7.5 (32H, m), 7.5-8.7 (4H, m).

(ii) Synthesis of Compound (8)

500 mg of Compound (8b) was treated in the same way as Example 1 (iii) to obtain 29 mg of Compound (8) (11% yield).

The identification data of Compound (8) were as follows.

$IR\nu_{max}^{Nujol}$ $cm^{-1}$: 3260 (sh), 1766, 1710, 1660, 1624, 1584, 1004, 800, 721, 671.

NMR δ(DMSO-$d_6$) ppm: 3.38, 3.62 (2H, ABq, J = 18), 5.21 (1H, d, J = 4.5), 5.50 (2H, br. s), 5.80 (1H, d.d, J = 4.5, 9), 6.77 (1H, s), 7.6-8.8 (3H, m), 9.60 (1H, d, J = 9).

Example 9

Synthesis of 7β-[2-(2-aminothiazol-4-yl)-2-syn-hydroxyiminoacetamido]-3-[[3-(2-aminothiazol-4-yl)-1-pyridinio]methyl]-3-cephem-4-carboxylic acid chloride (Compound (9))

(i) Synthesis of Compound (9b) (p-methoxybenzyl 7β-[2-(2-tritylaminothiazol-4-yl)-2-syn-trityloxyiminoacetamido]-3-[[3-(2-aminothiazol-4-yl)-1-pyridinio]methyl]-3-cephem-4-carboxylate iodide)

886 mg of Compound (1c) was subjected to the same treatments as in Example 6 (i) by using 260 mg of NaI and 153 mg of 3-(2-aminothiazol-4-yl)pyridine to obtain 1.05 g of Compound (9b) (94% yield).

The identification data of Compound (9b) were as follows.

$IR\nu_{max}^{Nujol}$ $cm^{-1}$: 3372, 1787, 1718, 1672, 1611, 1245, 1218, 1178, 1029, 964, 827, 754, 702, 636.

NMR δ(DMSO-$d_6$) ppm: 3.30, 3.57 (2H, ABq, J = 18), 3.67 (3H, s), 5.17 (1H, d, J = 4.5), 5.20 (2H, s), 5.50, 5.63 (2H, ABq, J = 13), 5.81 (1H, d.d, J = 4.5, 9), 6.43 (1H, s), 6.77 (2H, d, J = 8.5), 6.7 = 7.5 (34H, m), 7.40 (1H, s), 7.7-9.3 (4H, m), 9.66 (1H, d, J = 9).

(ii) Synthesis of Compound (9)

950 mg of Compound (9b) was treated according to the same procedure as Example 1 (iii) to obtain 109 mg of Compound (9) (25% yield).

The identification data of Compound (9) were as follows.

$IR\nu_{max}^{Nujol}$ $cm^{-1}$: 3200 (sh), 1773, 1627, 1055, 1011, 804, 722, 671.

NMR δ(DMSO-$d_6$) ppm: 3.43, 3.62 (2H, ABq, J = 17), 5.20 (1H, d, J = 4.5), 5.56, 5.68 (2H, ABq, J = 14), 5.78 (1H, d.d, J = 4.5, 9), 6,75 (1H, s), 7.55 (1H, s), 7.8-9.9 (5H, m).

Example 10

Synthesis of 7β-[2-(2-aminothiazol-4-yl)-2-syn-hydroxyiminoacetamido)-3-[[3-(2-methylaminothiazol-4-yl)-1-pyridinio]methyl]-3-cephem-4-carboxylic acid chloride (Compound (10))

(i) Synthesis of Compound (10) (p-methoxybenzyl 7β-[2-(2-tritylaminothiazol-4-yl)-2-syn-trityloxyiminoacetamido]-3-[[3-(2-methylaminothiazol-4-yl)-1-pyridinio]methyl]-3-cephem-4-carboxylate iodide)

511 mg of Compound (1c) was treated according to the same procedure as Example 6 (i) by using 450

mg of NaI and 96 mg of 3-(2-methylaminothiazol-4-yl)pyridine to obtain 555 mg of Compound (10b) (85% yield).

The identification data of Compound (10b) were as follows.

IR$\nu_{max}$ $^{Nujol}$ $cm^{-1}$: 3362, 3253, 1787, 1718, 1676, 1612, 1246, 1220, 1176, 1156, 1029, 963, 825, 753, 702.

NMR δ(DMSO-$d_6$ + $CD_3OD$) ppm: 2.87 (3H, S), 3.52 (2H, br. S), 3.66 (3H, S), 5.17 (3H, br. S), 5.53 (2H, br, S), 5.78 (1H, d, J = 4.5), 6.43 (1H, S), 6.72 (2H, d, J = 8.5, 6.8-7.8 (32H, m), 7.34 (1H, S), 7.98 (1H, m), 8.66 (2H, m), 9.16 (1H, br. S).

(ii) Synthesis of Compound (10)

508 mg of Compound (10b) was treated according to the same procedure as Example 1 (iii) to obtain 100 mg of Compound (10) (16% yield).

The identification data of Compound (10) were as follows.

IR$\nu_{max}$ $^{Nujol}$ $cm^{-1}$: 3300 (sh), 1772, 1709, 1618, 1046, 1004, 716, 664.

NMR δ(DMSO-$d_6$) ppm: 2.93 (3H, S), 3.47, 3.61 (2H, ABq, J = 18), 5.21 (1H, d, J = 4.5), 5.54, 5.76 (2H, ABq, J = 13), 5.93 (1H, d.d, J = 4.5, 9), 6.76 (1H, S), 7.55 (1H, S), 8.05 (1H, m), 8.7-9.0 (2H, m), 9.51 (1H, br.S).

Example 11

Synthesis of 7β-[2-(2-aminothiazol-4-yl)-2-syn-hydroxyiminoacetamido]-3-[[3-(2-methylthiazol-4-yl)-l-pyridinio]methyl]-3-cephem-4-carboxylic acid chloride (Compound (11))

(i) Synthesis of Compound (11b) (p-methoxybenzyl 7β-[2-(2-tritylaminothiazol-4-yl)-2-syn-trityloxyiminoacetamido]-3-[[3-(2-methylthiazol-4-yl)-1-pyridinio]methyl]-3-cephem-4-carboxylate iodide)

1.451 g of Compound (1c) was treated according to the procedure of Example 1 (ii) by using 639 mg of NaI and 250 mg of 3-(2-methylthiazol-4-yl)pyridine to obtain 1.52 g of Compound (11b) (83% yield).

The identification data of Compound (11b) were as follows.

IR$\nu_{max}$ $^{Nujol}$ $cm^{-1}$: 3365, 1787, 1715, 1678, 1612, 1584, 1246, 1175, 1031, 964, 828, 753, 700, 633.

NMR δ($CDCl_3$) ppm: 2.66 (3H, S), 3.67 (2H, br. S), 3.70 (3H, S), 5.03 (1H, d, J = 4.5), 5.18 (2H, S), 5.66, 5.91 (2H, ABq, J = 14), 6.03 (1H, d.d., J = 4.5, 9), 6.33 (1H, S), 6.75 (2H, d, J = 9), 6.7-7.6 (33H, m), 8.18 (1H, m), 8.33 (1H, S), 8.80 (1H, m), 10.12 (1H, br, s).

(ii) Synthesis of Compound (11)

1.42 g of Compound (11b) was treated in a similar manner as Example 1 (iii) to obtain 127 mg of Compound (11) (21% yield).

The identification data of Compound (11) were as follows.

IR$\nu_{max}$ $^{Nujol}$ $cm^{-1}$: 3200 (sh), 1773, 1661, 1628, 1178, 1148, 1054, 1010, 721.

NMR δ(DMSO-$d_6$ + $CD_3OD$) ppm: 2.74 (3H, S), 3.30, 3.60 (2H, ABq, J = 18), 5.18 (1H, d, J = 4.5), 5.53, 5.67 (2H, ABq, J = 14), 5.78 (1H, d, J = 4.5), 6.79 (1H, S), 8.27 (1H, S), 7.8-9.7 (4H, m).

Example 12

Synthesis of 7β-[2-(2-aminothiazol-4-yl)-2-syn-hydroxyiminoacetamido]-3-[[4-(2-methylaminothiazol-4-yl)-1-pyridinio]methyl]-3-cephem-4-carboxylic acid chloride (Compound (12))

(i) Synthesis of Compound (12b) (p-methoxybenzyl 7β-[2-(2-tritylaminothiazol-4-yl)-2-syn-trityloxyiminoacetamido]-3-[[4-(2-methylaminothiazol-4-yl)-1-pyridinio]methyl]-3-cephem-4-carboxylate iodide)

1.022 g of Compound (1c) was treated according to the procedure of Example 6 (i) by using 195 mg of NaI and 142 mg of 4-(2-methylaminothiazol-4-yl)pyridine to obtain 859 mg of Compound (12b) (68% yield).

The identification data of Compound (12b) were as follows.

IR$\nu_{max}$ KBr $cm^{-1}$: 3025, 2961, 1787, 1715, 1674, 1629, 1566, 1289, 1241, 1212, 1174, 1154, 1028, 958, 753, 698.

NMR δ($CDCl_3$ + $CD_3OD$) ppm: 2.94 (3H, S), 3.72 (5H, br. S), 5.09 (1H, d, J = 5.0), 5.20 (2H, S), 5.56 (2H, br. S), 5.95 (1H, d, J = 5.0), 6.42 (1H, S), 6.80 (2H, d, J = 8.2), 7.13 (30H, m), 7.27 (2H, d, J = 8.2), 7.56 (1H, S), 8.15 (2H, d, J = 6.4), 8.85 (2H, d, J = 6.4).

(ii) Synthesis of Compound (12)

441 mg of Compound (12b) was treated in accordance with Example 1 (iii) to obtain 32 mg of Compound (12) (16.5% yield).

The identification data of Compound (12) were as follows.

IR$\nu_{max}$ KBr $cm^{-1}$: 3380, 1766, 1628, 1568, 1526, 1386, 1388, 1148, 1057.

NMR δ(DMSO-$d_6$ + $CD_3OD$) ppm: 2.90 (3H, s), 3.68 (2H, br. S), 5.03 (1H, d, J = 4.6), 5.12 (1H, d, J = 14), 5.51 (1H, d, J = 14), 5.67 (1H, d, J = 4.6), 6.54 (1H, S), 7.82 (1H, S), 8.19 (2H, d, J = 6.4), 8.94 (2H, d, J = 6.4).

Example 13

Synthesis of 7β-[2-(2-aminothiazol-4-yl)-2-syn-hydroxyiminoacetamido]-3-[[4-(2-methylthiazol-4-yl)-1-pyridinio]methyl]-3-cephem-4-carboxylic acid chloride (Compound (13))

(i) Synthesis of Compound (13b) (p-methoxybenzyl 7β-[2-(2-tritylaminothiazol-4-yl)-2-syn-trityloxyiminoacetamido]-3-[[4-(2-methylthiazol-4-yl)-1-pyridinio]methyl]-3-cephem-4-carboxylate iodide)

1,098 mg of p-methoxybenzyl 7β-[2-(2-tritylaminothiazol-4-yl)-2-syn-trityloxyiminoacetamido]-3-iodomethyl-3-cephem-4-carboxylate and 159 mg of 4-(2-methylthiazol-4-yl)pyridine were dissolved in 6 ml of acetone, and the mixture was stirred at room temperature for 3.5 hours. Then acetone was concentrated under reduced pressure, and the mixture was washed with ethyl acetate and then added with isopropyl alcohol to cause separating of crystals, and the crystals were filtered out and dried in vacuo to obtain 45 mg of Compound (13b) (39.2% yield).

The identification data of Compound (13b) were as follows.

IR$\nu_{max}$ KBr $cm^{-1}$: 1787, 1718, 1675, 1632, 1243, 1174, 1028, 960, 825, 754, 700, 633.

NMR δ($CDCl_3$ + $CD_3OD$) ppm: 2.77 (3H, S), 3.73 (2H + 3H, br. S), 5.16 (1H, d, J = 5), 5.23 (2H, S), 5.56 (2H, br. S), 5.94 (1H, d, J = 5), 6.49 (1H, S), 6.82 (2H, d, J = 9), 7.20 (30H + 2H, S), 8.40 (2H, d, J = 7), 8.40 (1H, S), 8.92 (2H, d, J = 7).

(ii) Synthesis of Compound (13)

1.05 g of Compound (13b) was treated in the same way as Example 1 (iii) to obtain 137 mg of Compound (13) (33% yield).

The identification data of Compound (13) were as follows.

IR$\nu_{max}$ KBr $cm^{-1}$: 1768, 1635, 1533, 1350.

NMR δ(DMSO-$d_6$ + $CD_3OD$) ppm: 2.75 (3H, S), 3.71 (2H, br. S), 5.11 (1H, d, J = 4), 5.50 (2H, br. S), 5.74 (1H, d, J = 4), 6.60 (1H, S), 8.45 (2H, d, J = 6), 8.69 (1H, S), 9.23 (2H, d, J = 6).

Example 14

Synthesis of 7β-[2-(2-aminothiazol-4-yl)-2-syn-hydroxyiminoacetamido]-3-[(3-N,N-diethylcarbamoyl-l-pyridinio)methyl]-3-cephem-4-carboxylic acid chloride (Compound (14))

(i) Synthesis of Compound (14b) (p-methoxybenzyl 7β-[2-(2-tritylaminothiazol-4-yl)-2-syn-trityloxyiminoacetamido]-3-[(3-N,N-diethylcarbamoyl-1-pyridinio)methyl]-3-cephem-4-carboxylate iodide)

2.67 g of p-methoxybenzyl 7β-[2-(2-tritylaminothiazol-4-yl)-2-syn-trityloxyiminoacetamido]-3-iodomethyl-3-cephem-4-carboxylate and 650 mg of 3-N,N-diethylcarbamoylpyridine were dissolved in 15 ml of acetonitrile, and the mixture was stirred at room temperature for 4 hours and then treated in the same way as in example 13 (i) to obtain 2.02 g of Compound (14b) (65% yield).

The identification data of Compound (14b) were as follows.

IR$\nu_{max}$ KBr $cm^{-1}$: 1787, 1710, 1673, 1632, 1243, 1212, 1173, 1028, 960, 824, 752, 700, 634.

NMR δ($CDCl_3$ + $CD_3OD$) ppm: 1.17 (3H, t, J = 6), 1.23 (3H, t, J = 6), 3.2-3.7 (4H + 2H, m), 3.74 (3H, S), 5.11 (1H, d, J = 5), 5.20 (2H, S), 5.63 (3H, br, S), 5.91 (1H, d, J = 5), 6.45 (1H, S), 6.81 (2H, d, J = 9), 7.13 (30H + 2H, br. S), 7.9-8.6 (2H, m), 9.04 (2H, br. S).

(ii) Synthesis of Compound (14)

The procedure of Example 1 (iii) was followed except for use of 1.00 g of Compound (14b) to obtain 115 mg of Compound (14) (28% yield).

The identification data of Compound (14) were as follows.

IR$\nu_{max}$ KBr $cm^{-1}$: 1778, 1622, 1440, 1190, 1008.

NMR δ(DMSO-$d_6$ + $CD_3OD$) ppm: 0.9-1.4 (6H, m), 2.9-3.8 (4H + 2H, m), 5.10 (1H, d, J = 4), 5.35 (2H, br. s), 5.74 (1H, d, J = 4), 6.61 (1H, S), 8.0-9.6 (4H, m).

Referential Example 1

Synthesis of 3-(2-methylaminothiazol-4-yl)pyridine

4 g of 3-bromoacetylpyridine hydrobromide was dissolved in a mixed solution of 28 ml of tetrahydrofuran and 20 ml of water. To this solution were added 4.67 g of sodium acetate and then 2.56 g of N-methylthiourea with stirring under ice cooling, followed by stirring at the same temperature for 10 minutes and then at room temperature for 3 hours. Then the solution was added into ice-cold water and made alkaline with $NaHCO_3$. The aqueous mixture was extracted with ethyl acetate and the extract was washed with a saline solution and dried over $Na_2SO_4$. Then the solvent was distilled away and the residue was recrystallized from hydrous ethanol to obtain 3.4 g of the object compound.

The identification data of this compound were as follows.

IR$\nu_{max}$ $^{Nujol}$ $cm^{-1}$: 3217, 3110, 1586, 1571, 1059, 1021, 813, 721, 711.

NMR δ(DMSO-$d_6$) ppm: 2.87 (3H, d, J = 5), 4.06 (1H, S), 4.24 (1H, d.d, J = 4.5, 8), 7.44 (1H, d, J = 5), 7.99 (1H, d.t, J = 8, 2), 8.28 (1H, d.d., J = 4.5, 2), 8.88 (1H, d, J = 2).

Referential Example 2

Synthesis of 4-(2-methylaminothiazol-4-yl)pyridine

The process of Referential Example 1 was repeated by using 4-bromoacetylpyridine hydrobromide and N-methylthiourea to obtain the object compound.

The identification data of this compound were as follows.

IR$\nu_{max}$ $^{KBr}$ $cm^{-1}$: 3207, 2965, 1597, 1571, 1404, 1338, 1297, 1237, 1226, 1202, 1154, 1057, 1000, 831, 706, 659, 564.

NMR δ(DMSO-$d_6$ + $CDCl_3$) ppm: 2.90 (3H, d, J = 4.8), 7.07 (1H, S), 7.37 (1H, d, J = 4.8), 7.62 (2H, d.d, J = 1.8, 4.4), 8.42 (2H, d.d, J = 1.8, 4.4).

Referential Example 3

Synthesis of 3-(2-methylthiazol-4-yl)pyridine

3.9 g of 3-bromoacetylpyridine hydrobromide was refluxed under heating in 100 ml of methanol by adding 1.82 g of thioacetamide for one hour. Methanol was concentrated, and after adding water, the solution was made alkaline with $Na_2SO_4$ and extracted with chloroform. The extract was dried over $Na_2SO_4$ and concentrated, and the residue was added with benzene and cyclohexane to cause separating of crystals. The crystals were filtered out to obtain 1.3 g of the object compound.

The identification data of this compound were as follows.

IR$\nu_{max}$ $^{KBr}$ $cm^{-1}$: 1685, 1574, 1495, 1406, 1171, 1023, 854, 812, 740, 705.

NMR δ($CDCl_3$-$d_6$ + $CD_3OD$) ppm: 2.73 (3H, S), 7.32 (1H, d.d, J = 8, 5), 7.43 (1H, S), 8.14 (1H, d.t, J = 8, 1), 8.43 (1H, d.d, J = 5, 1.5), 8.98 (1H, d, J = 1.5).

Test Example 3 (Test on antibacterial potency against standard strains)

The minimum growth inhibitory concentration was determined for each of the 21 standard strains shown in Table 3, according to the same method as used in Test Example 1. The results are shown Table 3.

In the test, FMOX (flomoxef), CTM (cefotiam) and CEZ (cefazolin) were used as control agents.

Table 3: Antibacterial Potency Against Standard Strains

(MIC: µg/ml)

| Bacterial Strains Tested | Test Agents | | | | | |
|---|---|---|---|---|---|---|
| | Compd. (6) | Compd. (7) | Compd. (8) | Compd. (9) | Compd. (10) | Compd. (11) |
| S. aureus 209p JC-1 | 0.39 | 0.2 | 0.78 | 0.2 | 0.39 | 0.2 |
| S. aureus Smith | 0.78 | 0.39 | 1.56 | 0.2 | 0.78 | 0.39 |
| S. aureus 167 | 12.5 | 12.5 | 50 | 3.13 | 12.5 | 3.13 |
| S. aureus 195 | 3.13 | 3.13 | 25 | 1.56 | 3.13 | 1.56 |
| S. epidemidis IAM1296 | 0.2 | 0.1 | 0.39 | 0.05 | 0.2 | 0.1 |
| M. luteus ATCC 9341 | 0.1 | 0.1 | 0.1 | 0.05 | 0.1 | 0.05 |
| B. subtilis ATCC6633 | 0.78 | 0.39 | 0.39 | 0.2 | 0.39 | 0.2 |
| E. coli NIHJ JC-2 | 0.39 | 0.2 | 0.1 | 0.2 | 1.56 | 0.39 |
| E. coli CSH2 (RK1) | 1.56 | 0.78 | 0.39 | 0.39 | 3.13 | 0.78 |
| E. coli CSH2 (RE45) | 0.78 | 0.2 | 0.2 | 0.39 | 1.56 | 0.39 |
| K. pneumoniae IFO3317 | 0.39 | 0.2 | 0.1 | 0.2 | 0.39 | 0.2 |
| K. pneumoniae No. 42 | 0.78 | 0.39 | 0.39 | 0.78 | 6.25 | 0.78 |
| P. mirabilis IFO3849 | 0.39 | 0.2 | 0.1 | 0.39 | 3.13 | 0.78 |
| P. vulugaris OX-19 | 0.78 | 0.39 | 0.2 | 0.2 | 0.39 | 0.2 |
| M. Morganii IFO3848 | 0.2 | 0.1 | 0.05 | 0.1 | 0.2 | 0.1 |
| S. marcescens IAM1184 | 0.39 | 0.2 | 0.39 | 0.39 | 3.13 | 0.39 |
| E. cloacae ATCC13407 | 1.56 | 0.39 | 1.56 | 0.78 | 12.5 | 3.13 |
| C. freundii ATCC8090 | 0.39 | 0.2 | 0.39 | 0.2 | 1.56 | 0.39 |
| P. aeruginosa NCTC10490 | 3.13 | 1.56 | 100 | 3.13 | 25 | 6.25 |
| A. faecalis ATCC3750 | 3.13 | 1.56 | 0.78 | 1.56 | 6.25 | 3.13 |
| A. calcoaceticos T59 | 12.5 | 3.13 | 25 | 12.5 | 50 | 25 |

Table 3 (Continued)

| Bacterial Strains Tested | Test Agents | | | | | |
|---|---|---|---|---|---|---|
| | Compd. (12) | Compd. (13) | Compd. (14) | FMPX | CTM | CEZ |
| S. aureus 209p JC-1 | 0.39 | 0.2 | 0.78 | 0.39 | 0.39 | 0.2 |
| S. aureus Smith | 0.39 | 0.39 | 0.78 | 0.39 | 0.39 | 0.2 |
| S. aureus 167 | 5.25 | 6.25 | 12.5 | 50 | 100 | 100 |
| S. aureus 195 | 3.13 | 3.13 | 6.25 | 3.13 | >100 | 50 |
| S. epidemidis IAM1296 | 0.2 | 0.2 | 0.39 | 0.2 | 0.2 | 0.1 |
| M. luteus ATCC9341 | 0.05 | 0.05 | 0.2 | 0.05 | 0.39 | 0.78 |
| B. subtilis ATCC6633 | 0.2 | 0.2 | 0.39 | 0.2 | 0.2 | 0.2 |
| E. coli NIHJ JC-2 | 0.2 | 0.2 | 0.78 | 0.05 | 0.1 | 1.56 |
| E. coli CSH2 (RK1) | 0.39 | 0.2 | 3.13 | 0.05 | 0.1 | 3.13 |
| E. coli CSH2 (RE45) | 0.2 | 0.2 | 0.78 | 0.05 | 0.1 | 1.56 |
| K. pneumoniae IFO3317 | 0.1 | 0.1 | 0.78 | 0.025 | 0.05 | 0.78 |
| K. pneumoniae No. 42 | 0.78 | 0.78 | 3.13 | 0.05 | 0.39 | 3.13 |
| P. mirabilis IFO3849 | 0.78 | 0.39 | 1.56 | 0.2 | 0.39 | 6.25 |
| P. vulugaris OX-19 | 0.1 | 0.2 | 0.78 | 0.39 | 0.39 | 12.5 |
| M. Morganii IFO3848 | 0.05 | 0.05 | 0.39 | 0.39 | 0.1 | 25 |
| S. marcescens IAM1184 | 0.78 | 0.39 | 0.78 | 0.2 | 1.56 | >100 |
| E. cloacae ATCC13407 | 1.56 | 0.78 | 3.13 | 25 | 3.13 | >100 |
| C. freundii ATCC8090 | 0.39 | 0.2 | 0.78 | 0.39 | 0.78 | 3.13 |
| P. aeruginosa NCTC10490 | 12.53 | 12.5 | 6.25 | >100 | >100 | >100 |
| A. faecalis ATCC3750 | 1.56 | 1.56 | 3.13 | 0.1 | 1.56 | 12.53 |
| A. calcoaceticos T59 | 50 | 50 | 12.5 | 50 | 100 | >100 |

Test Example 4 (Test on antibacterial potency against clinically isolated MRSA)

MIC 50 and MIC 90 were determined for each of the 52 strains of clinically isolated MRSA according to the same method as used in Test Example 2. The results are shown in Table 4.

VCM (vancomycin) was used as control agent.

Table 4: Antibacterial Potency Against Clinically isolated MRSA

| Test Agents | Antibacterial Potency | |
|---|---|---|
| | MIC50 | MIC90 |
| VCM | 0.74 | 1.38 |
| Compd. (6) | 2.15 | 7.18 |
| Compd. (7) | 2.04 | 5.44 |
| Compd. (8) | 8.34 | 37.89 |
| Compd. (9) | 1.48 | 2.92 |
| Compd. (10) | 4.04 | 8.37 |
| Compd. (11) | 1.85 | 3.04 |
| Compd. (12) | 1.86 | 4.61 |
| Compd. (13) | 2.21 | 5.51 |
| Compd. (14) | 6.77 | 11.79 |

As has become apparent from the foregoing, novel antibacterial agents, especially ones effective against MRSA, have been provided by the present invention.

## Claims

1. Cephem compounds represented by the following general formula (I):

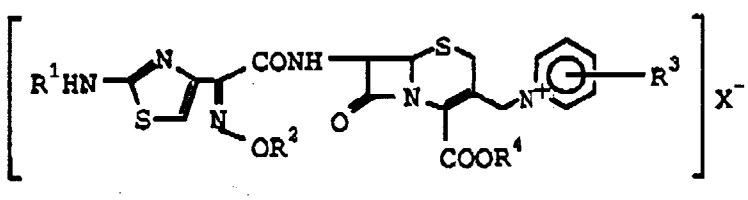

(I)

wherein $R^1$ is a hydrogen atom or a protective group of the amino group; $R^2$ is a hydrogen atom or a protective group of the hydroxyimino group; $R^3$ is an alkyl group which may have appropriate substituent(s), a hydroxyl group which may have an appropriate substituent, a mecarpto group which may have appropriate substituent(s), a cyano group, an acetyl group, an amino group which may have appropriate substituent(s), a carbamoyl group which may have appropriate substituent(s), an ureido group or a heterocyclic group which may have appropriate substituent(s); $R^4$ is a hydrogen atom, a protective group of the carboxyl group or an anion (when $R^4$ is an anion, $X^-$ is excluded), and $X^-$ represents the acid radical anion of an inorganic acid or an organic acid.

2. The compounds set forth in Claim 1, wherein $R^3$ is represented by the following formula (1):

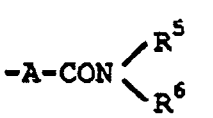

(1)

wherein A denotes a single bond, $-CH_2-$, -O-, -S-, -NH- or the like, and $R^5$ and $R^6$ represent independently a hydrogen atom or an alkyl group.

3. The compounds set forth in Claim 1, wherein $R^3$ is represented by the following formula (2):

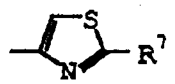

(2)

wherein $R^7$ is an alkyl group or an amino group which may have appropriate substituent(s).

4. Antibacterial agents containing as an active ingredient at least one cephem compound represented by the above general formula (I).

5. Anti-MRSA antibacterial agents containing as an active ingredient at least one cephem compound represented by the above general formula (I).

6. A compound according to any one of claims 1 to 3 for pharmaceutical use.

7. The use of a compound of any one of claims 1 to 3 for the manufacture of an anti-bacterial agent.

8. A use according to claim 7 wherein the anti-bacterial agent is an anti-MRSA agent.

9. A pharmaceutical composition comprising a compound according to any one of claims 1 to 3 and a pharmaceutically acceptable excipient diluent or carrier.

10. A method for the production of a compound of any one of claims 1 to 3 comprising at least one of the following:
(A) reaction of a compound of formula (II) wherein Z is a leaving group, preferably iodine, and $R^1$, $R^2$ and $R^4$ are as defined in claim 1 with a compound of formula III wherein $R^3$ is ad defined in claim 1

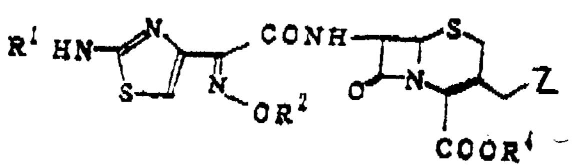

(II)

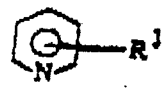

(III)

(B) reaction of a compound of formula (V) with a compound of formula (VI) wherein $R^1$, $R^2$, $R^3$, $R^4$ and X are as defined in claim 1.

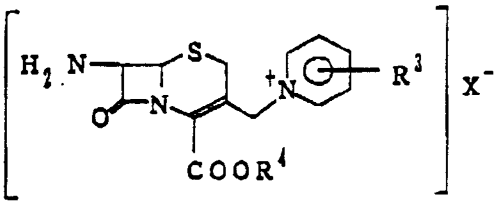

(V)

+

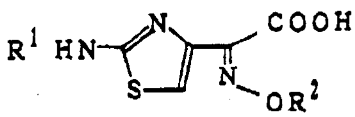

(VI)

(c) deprotecting a compound of formula (I) in which $R^1$ is a protecting group to form a compound of formula (I) in which $R^1$ is a hydrogen atom.

Fig. 1

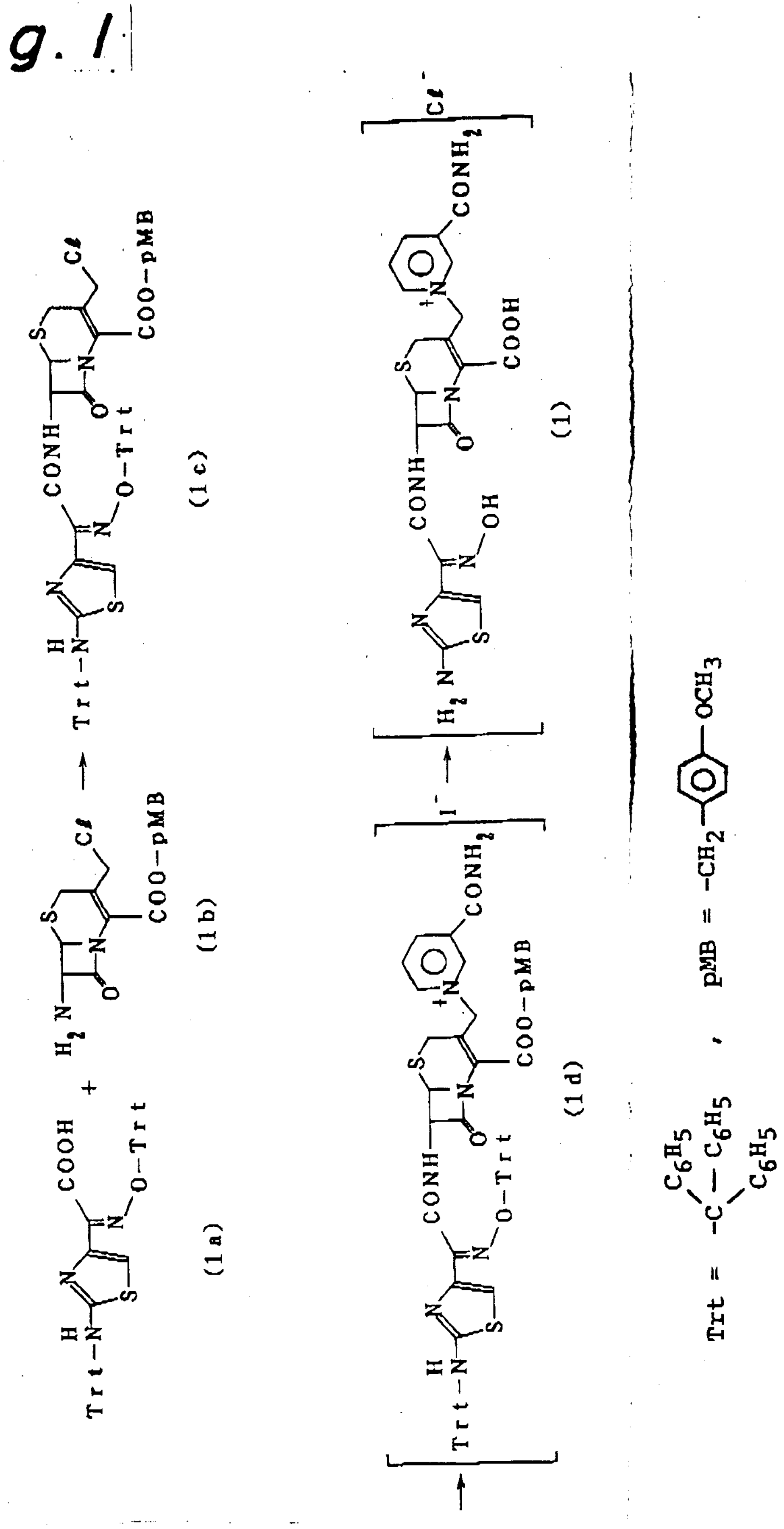

(1a) (1b) (1c) (1d) (1)

Fig. 2

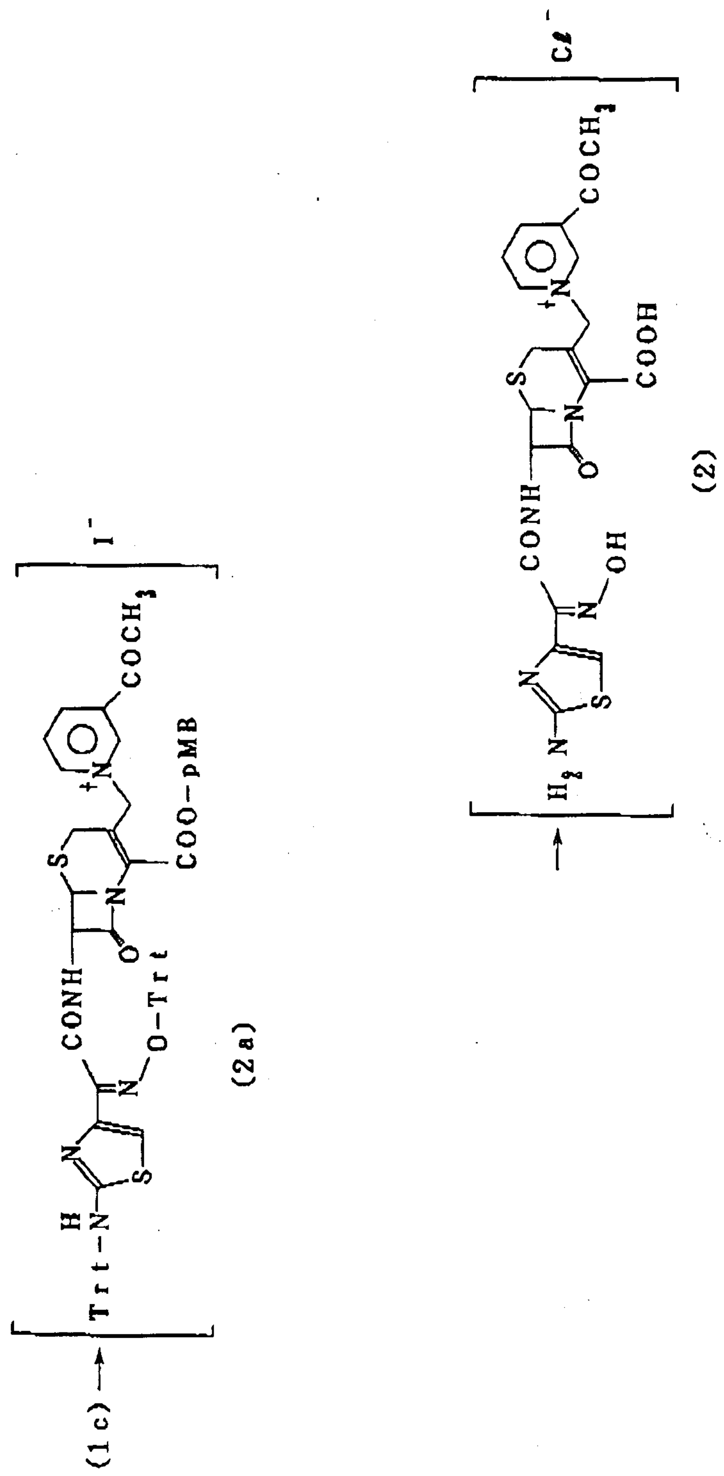

(1c)

(2a)

(2)

Fig.3

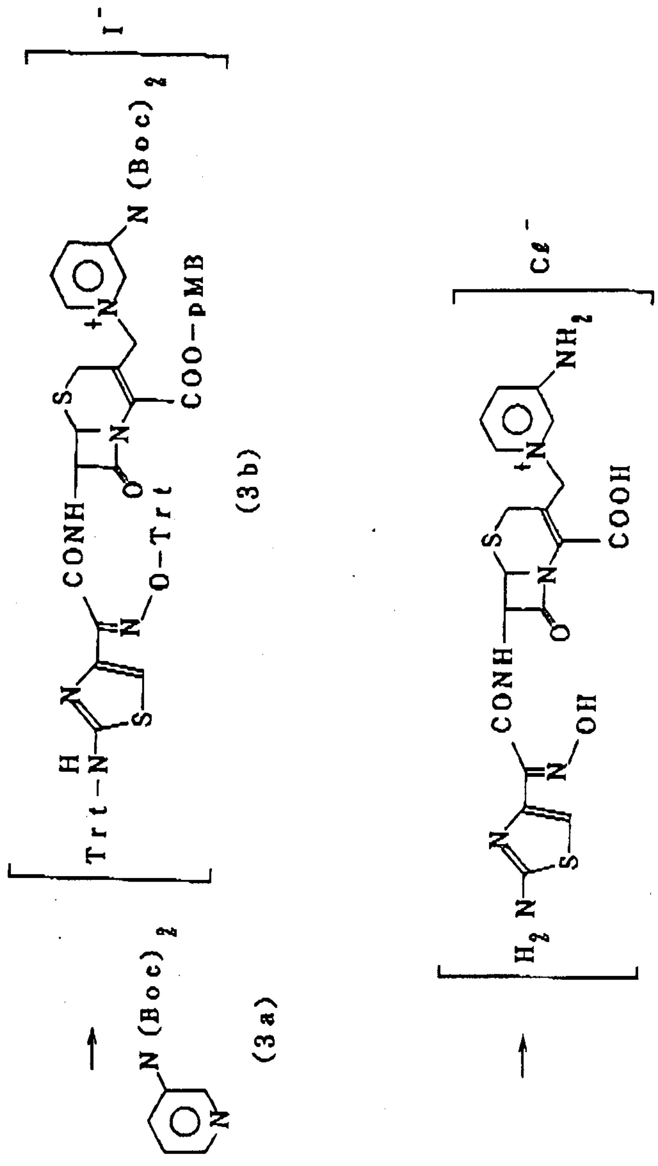

(1c)

(3a)

(3b)

(3)

*Fig.4*

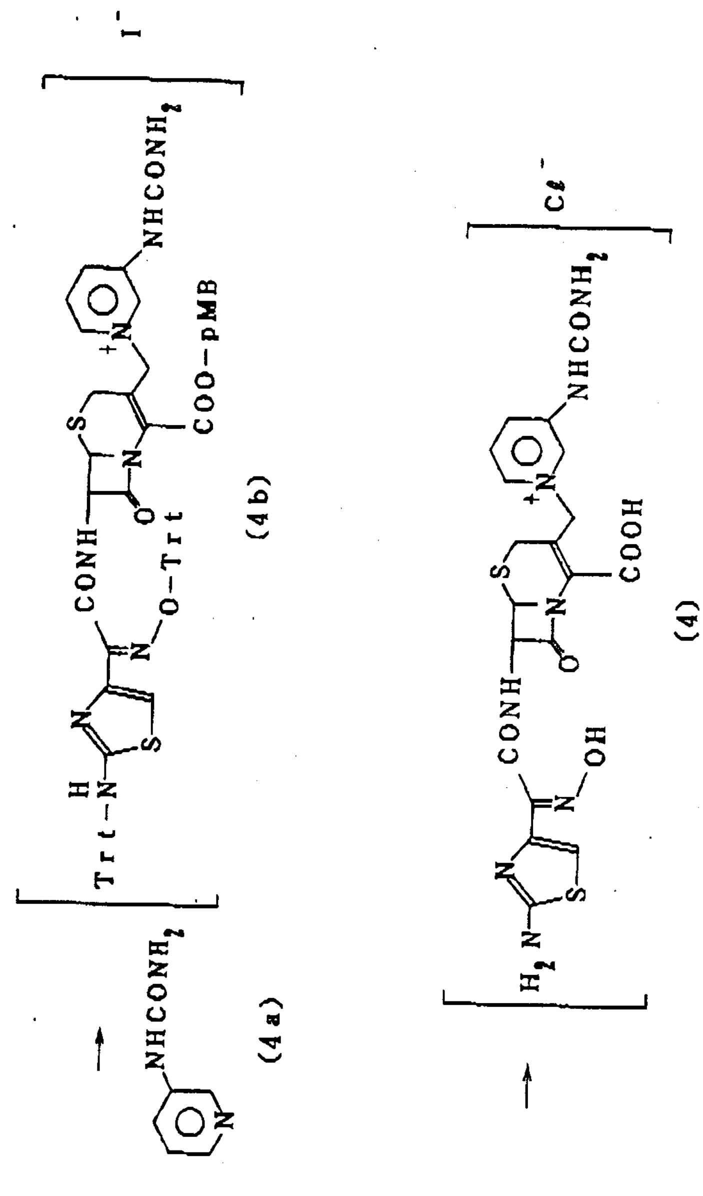

(1c) (4a) (4b) (4)

Fig. 5

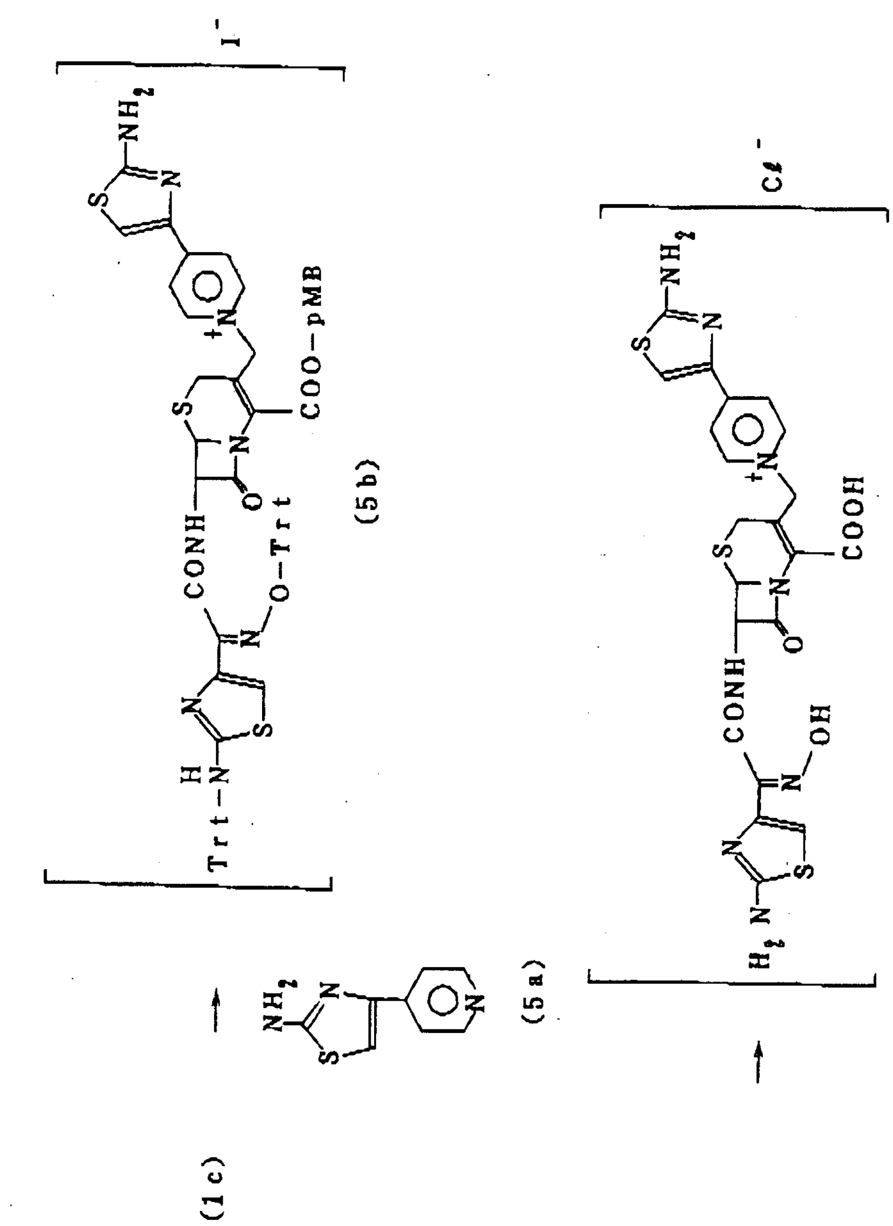

(1c) (5a) (5b) (5)